(19) Europäisches Patentamt
European Patent Office
Office européen des brevets

(11) **EP 0 799 598 A1**

(12) **EUROPÄISCHE PATENTANMELDUNG**

(43) Veröffentlichungstag:
08.10.1997 Patentblatt 1997/41

(51) Int. Cl.$^6$: **A61B 5/00**

(21) Anmeldenummer: 96118305.0

(22) Anmeldetag: 15.11.1996

(84) Benannte Vertragsstaaten:
AT BE CH DE DK ES FI FR GB GR IE IT LI LU MC NL PT SE

(30) Priorität: 02.04.1996 DE 19613225

(71) Anmelder: Barnikol, Wolfgang, Prof. Dr.Dr.
D-55128 Mainz (DE)

(72) Erfinder:
• Barnikol, Wolfgang, Prof. Dr. Dr.
55128 Mainz (DE)
• Niehoff, Klaus
55127 Mainz (DE)

(74) Vertreter: Beil, Hans Christoph, Dr. et al
Hansmann & Vogeser,
Patent- und Rechtsanwälte,
Postfach 80 01 40
65901 Frankfurt (DE)

(54) **Messung des Partialdruckes und der Leitfähigkeit von Sauerstoff in lebendem Gewebe**

(57) Vorrichtung zur Messung des Sauerstoff-Partialdruckes von Gewebe bei gleichzeitiger Ermittlung der Sauerstoffleitfähigkeit des betreffenden Gewebes an der Meßstelle, bestehend aus einem Meßkopf (1) mit einerseits einer Anordnung (15-20) zur Bestimmung der Lumineszenz-Löschung eines durch Licht angeregten Fluoreszenz-Farbstoffs und andererseits einer elektrochemischen Kette aus Blei (9) und Silber (12) in KOH (14).

Fig. 1

EP 0 799 598 A1

**Beschreibung**

Gegenstand der Erfindung ist eine Vorrichtung zur epigeweblichen Messung des intrageweblichen Sauerstoff-Partialdrucks von Geweben bei gleichzeitiger Ermittlung der Sauerstoffleitfähigkeit des betreffenden Gewebes an der Meßstelle.

Der Sauerstoff-Partialdruck eines peripheren Gewebes hat grundsätzliche Bedeutung als globaler Indikator für die regelrechte Funktion der geweblichen Versorgung des Organismus und damit auch des gesamten Sauerstofftransportsystems eines Menschen, an denen Lunge und Blutkreislauf maßgebend beteiligt sind. Diese Größe ist deshalb besonders wichtig für die Überwachung von Patienten und hat daher besondere Bedeutung für die Intensivmedizin und die Anästhesiologie. Aber auch wenn es um periphere Durchblutungsstörungen geht, besteht für den geweblichen Sauerstoffpartialdruck ein großes Interesse. Der Sauerstoff-Partialdruck der Haut hat hierbei besonderes Interesse gefunden, weil die Haut das am leichtesten zugängliche Organ ist und sie eine nicht-invasive Bestimmung der interessierenden Sauerstoff-Größen, nämlich des Partialdrucks und der Leitfähigkeit, ermöglicht.

Gerade die letztgenannte Größe spielt eine gewichtige Rolle für Diagnose und Prognose von Durchblutungsstörungen der Haut, weil diese, neuen Erkenntnissen zufolge, in entscheidender Weise von außen mit Sauerstoff versorgt werden kann. Gerade mit Hilfe der Sauerstoffleitfähigkeit der Haut lassen sich kutane Durchblutungsstörungen graduieren und eventuelle Therapieerfolge feststellen.

Das heute bevorzugte Verfahren der quantitativen Erfassung des sogenannten transkutanen Sauerstoff-Partialdrucks ist die polarographische Bestimmung des epikutanen Sauerstoff-Partialdrucks mit der (modifizierten) Elektrode nach Clark. Dabei wird die Haut unter der Elektrode erwärmt, um den epikutanen Sauerstoff-Partialdruck demjenigen der Haut oder gar dem arteriellen anzunähern. Dieses Verfahren hat insbesondere für die Intensiv-Überwachung Neugeborener Bedeutung erlangt. Wesentliche Nachteile dieser Methode sind der mit dem Meßwert starr gekoppelte Sauerstoffverbrauch, der dadurch bedingte systematische Fehler falsch zu niedriger Werte und die Notwendigkeit einer Erwärmung der Haut auf mindestens 43°C, um von der lokalen Durchblutung unabhängige Werte zu erhalten. Insbesondere im Falle Neugeborener führen solche Temperaturen nach etwa 2 Stunden zu Hautverbrennungen ersten Grades, so daß der Sensor auf der Haut immer wieder versetzt werden muß.

Aus DE 25 39 771A1 ist ein Meßkopf vom Clark-Typ zur Messung des Partialdrucks von Gasen bekannt, bei dem das Meßsystem von dem umgebenden, zu messenden Medium durch eine für die zu bestimmenden Gase durchlässige, für den Elektrolyten des Meßkopfes aber undurchlässige Trennschicht mit im einzelnen beschriebenen Eigenschaften getrennt ist. Der Einsatz dieses Meßkopfes mit Anoden, Kathoden und Elektrolyt zur Bestimmung der Sauerstoffleitfähigkeit oder dessen Kombination mit anderen Meßanordnungen wird nicht beschrieben.

Eine alternative Möglichkeit sind optische Meßverfahren, insbesondere die Lumineszenzlöschung durch Sauerstoff, wie sie beispielsweise aus DE 39 00 191A1 bekannt sind. Gegenüber elektrolytischen Methoden hat dieses Verfahren den Vorteil, daß kein Sauerstoff verbraucht wird. Eine Kombination mit einer Anordnung zur gleichzeitigen Ermittlung der Sauerstoffleitfähigkeit des betreffenden Gewebes ist dort aber nicht offenbart.

Neben dem innergeweblichen Sauerstoff-Partialdruck ist auch die Sauerstoff-Leitfähigkeit eines Gewebes von Interesse, wobei beide Messwerte vorzugsweise gemeinsam ermittelt werden sollten. Dies war mit den bisher bekannten Methoden nicht möglich.

Die erfindungsgemäße Vorrichtung löst dieses Problem, indem mittels der Lumineszenzlöschung einerseits der Sauerstoff-Partialdruck verbrauchsfrei - und somit frei von systematischen Fehlern - und unter Zuhilfenahme einer geeigneten elektrochemischen Kette andererseits die Sauerstoff-Leitfähigkeit des zu untersuchenden Gewebes bestimmt wird. Die Kette fungiert als Sauerstoffsenke, so daß der diffuse Sauerstoffstrom nach außen gerichtet ist.

Die erfindungsgemäße Vorrichtung besteht aus einem Meßkopf mit einem zentral angeordneten Lichtleiterschacht, einer in das untere Ende des Lichtleiterschachtes eingesetzten und diesen gasdicht verschließenden Glaslinse, auf der eine einlagige Schicht aus einem Fluoreszenz-Farbstoff tragenden feinkörnigen Material aufgebracht und durch eine hoch sauerstoffdurchlässige, lichtundurchlässige Deckmembran abgedeckt ist, mehreren konzentrisch zu dem Lichtleiterschacht in Anodenschächten angeordneten Anoden, die über Elektrolytbrücken am unteren Ende des Meßkopfes eine elektrolytisch leitende Verbindung zur Kathode haben, mehreren konzentrisch zu dem Lichtleiterschacht in einer Kontaktfläche am unteren Ende des Meßkopfes endenden Kathodenzuleitungen, die mit einer Kathodenfläche verbunden sind, die ihrerseits von einer den unteren Teil des Meßkopfs umschließenden hochsauerstoff-durchlässigen Meßflächen-Membran getragen wird, wobei zwischen Meßkopf und Membran eine alkalische Lösung eingeschlossen ist, und einem in dem Lichtleiterschacht angeordneten Y-Lichtleiter, an dessen Anregungsschenkel eine Lichtquelle und an dessen Detektionsschenkel ein Lichtmeßgerät angeordnet ist, und einem in dem die Anode und Kathode am oberen Ende des Meßkopfes verbindenden Stromkreis angeordneten Meßgerät.

Anhand der beigefügten Zeichnungen wird die Vorrichtung näher erläutert. Dabei zeigt

Fig. 1     die schematische Gestaltung der Meßeinrichtung

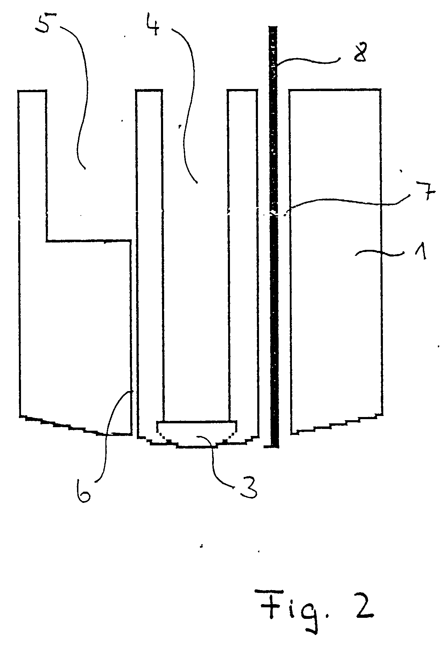

Fig. 2     einen schematischen Querschnitt durch einen Teil des Meßkopfes.

Der Meßkopf muß sauerstoffdicht, KOH-beständig, lichtundurchlässig und bis ca. 50°C temperaturbeständig sein. Ferner muß sein Material elektrisch und elektrolytisch inert sein. Vorzugsweise besteht der Meßkopf aus hartem Polyvinylchlorid. Der Meßkopf weist einen zentralen Schacht 4 zur Aufnahme des Y-Lichtleiters 2 auf. Am Ende des Lichtleiterschachts ist die Linse 3 sauerstoffdicht eingeklebt. Der Meßkopf weist ferner vier konzentrisch angeordnete Anodenschächte 6 auf, die sich in Bohrungen fortsetzen. Ferner weist der Meßkopf 4 ebenfalls konzentrisch angeordnete Kathodenbohrungen 7 zur Aufnahme der Kathodenzuleitungen auf.

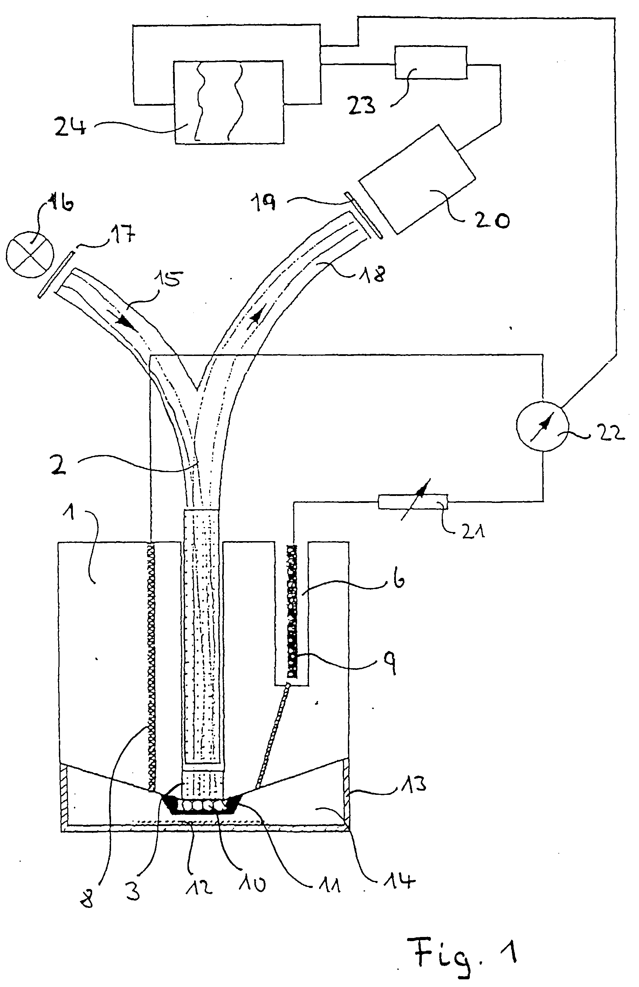

Wie aus Figur 1 ersichtlich, ist der Meßkopf in seinem unteren Ende durch eine sauerstoffdurchlässige Membran 13 abgeschlossen, die einerseits einen Raum 14 bildet und andererseits eine aus Silber bestehende Kathodenfläche 12 trägt. In den Anodenschächten 6 sind parallel geschaltete Anoden 9 angeordnet, die jeweils aus ca. 25 cm langen als Spirale gewickelten Bleidrähten bestehen. Die Anodenschächte stehen über einen in der Bohrung angeordneten Asbestdocht mit dem Raum 14 in Verbindung, der mit einer KOH-Lösung gefüllt ist.

In den Kathodenbohrungen 7 ist die Kathodenzuleitung 8 angeordnet, die in ihrem unteren Teil aus Silberblech besteht und an ihrem Ende einen rechtwinkelig abgeknickten Fuß bildet, der auf der Kathodenfläche 12 aufliegt.

Anode und Kathode sind mit einem Meßgerät 22 verbunden, wobei in dem Stromkreis zwischen Meßgerät und Anode ein regelbarer Widerstand 21 angeordnet sein kann. Die so gebildete elektrochemische Kette ist vollständig sauerstoffspezifisch: An der Silberkathode wird Sauerstoff reduziert, so daß nur bei Anwesenheit von Sauerstoff ein Stromfluß festzustellen ist. Aus der Stärke des gemessenen Elektrodenstroms läßt sich die Größe des Sauerstoffstroms berechnen und daraus die Sauerstoffleitfähigkeit ableiten.

Unter der Linse 3 ist eine Schicht aus einem auf einem inerten Träger angebrachten Lumineszenz-Farbstoff 10 angeordnet, der von einer sauerstoffdurchlässigen, aber lichtundurchlässigen schwarzen Silikonmembran 11 bedeckt ist.

Sowohl die Linse 3 wie auch die Kathodenzuleitungen und die als Elektrolytbrücken dienenden Asbestdochte müssen sauerstoffdicht in dem Meßkopf angebracht sein, damit auf diesem Wege kein Sauerstoff dem Raum 14 zugeführt werden kann.

Der in dem Lichtleiterschacht 4 angeordnete Y-förmige Lichtleiter 2 besteht aus einem Anregungsschenkel 15, vor dem eine geeignete Lichtquelle 16 sowie ein Filter 17 angeordnet sind. Vor dem Detektionsschenkel 18 ist ein für das Anregungslicht undurchlässiger Filter 19 und ein Fotovervielfacher 20 angeordnet. Über einen Vorverstärker 23 kann das Ausgangssignal des Fotovervielfachers ebenso wie das Ausgangssignal des Meßgeräts 22 einem Schreiber 24 zugeführt werden.

Der dargestellte erfindungsgemäße Meßkopf kann auf zweierlei Art zur Bestimmung des innergeweblichen Sauerstoff-Partialdrucks betrieben werden, nämlich entweder durch Vorgabe eines Sauerstoffstroms (Messung im Diffusionsmodus; stationärer Meßmodus) oder durch bloße Messung eines Sauerstoff-Partialdrucks nach Einstellung eines Druckausgleichs unter dem Meßdruck (statischer Meßmodus). In beiden Fällen dient das erste Fick'sche Diffusionsgesetz zur Beschreibung und Bestimmung der zu messenden Zustände der physiochemischen Vorgänge und zur Bestimmung des innergeweblichen Sauerstoffpartialdrucks sowie der Sauerstoffleitfähigkeit des unterhalb des Meßkopfes gelegenen Gewebes.

Das Ficksche Diffusionsgesetz, angewendet auf die hier beschriebene apparative Anordnung, stellt sich wie folgt dar:

$$MO_2 = LO_2 \times (icPO_2 - sPO_2) \tag{1}$$

Dabei sind

$MO_2$     diffuser Sauerstoffstrom nach außen, meßbar als Strom der elektrochemischen Kette
$LO_2$     Sauerstoffleitfähigkeit
$icPO_2$     innergeweblicher Sauerstoffpartialdruck
$sPO_2$     epikutaner Sauerstoffpartialdruck, bestimmbar mit dem Lumineszenzdetektor

Durch Umstellen der Gleichung (1) ergibt sich

$$sPO_2 = icPO_2 - MO_2 : LO_2 \tag{2}$$

Verschiedene Werte für $MO_2$ sind über die steuerbare Sauerstoffsenke (Widerstand 21) einstellbar; der zugehörige Wert $sPO_2$ wird gemessen. Daraus ergibt sich die Größe $icPO_2$ als Achsenabschnitt der meßbaren linearen Funktion und die Größe $LO_2$ als Kehrwert der Steigung. Messungen mit diesem Sensor erfordern kein Erwärmen der Haut.

Beispiel 1

Das Diagramm 1 gibt das Anwendungsbeispiel einer in vitro-Messung wieder. Hier wurde eine künstliche Membran mit ähnlicher Sauerstoffleitfähigkeit wie diejenige der Haut vor den Sensorkopf gespannt und gegen Luft gemessen. Die Messung belegt die Gültigkeit des Fickschen Gesetzes für die erfindungsgemäße Gestaltung, und die Größe icPO$_2$ gibt richtig den Sauerstoffpartialdruck der Luft wieder (1 µA entspricht 2,6 x 10$^{-6}$ µMol O$_s$/s).

Beispiel 2

Das Diagramm 2 repräsentiert eine Messung auf der Haut. Der hier ermittelte intrakutane Sauerstoffpartialdruck icPO$_2$ beträgt 44 mmHg.

sPO$_2$
(kPa)

icPO$_2$: 150 mmHg (Luft)

Diagr. 1

$\dot{M}O_2$ (µA)

sPO$_2$
(kPa)

icPO$_2$: 44 mmHg

Diagr. 2

$\dot{M}O_2$ (µA)

**Patentansprüche**

1. Vorrichtung zur epigeweblichen Messung des intrageweblichen Sauerstoff-Partialdruckes von Geweben bei gleichzeitiger Ermittlung der Sauerstoffleitfähigkeit des betreffenden Gewebes an der Meßstelle, bestehend aus

   a) einem Meßkopf (1) mit einem zentral angeordneten Lichtleiterschacht (4), einer in das untere Ende des Lichtleiterschachts (4) eingesetzten und diesen gasdicht verschließenden Glaslinse (3), auf der eine einlagige Schicht (10) aus einem Fluoreszenz-Farbstoff tragenden feinkörnigen Material aufgebracht und durch eine hoch sauerstoff-durchlässige, lichtundurchlässige Deckmembran (11) abgedeckt ist,

   mehreren konzentrisch zu dem Lichtleiterschacht (4) in Anodenschächten (6) angeordneten Anoden (9), die über Elektrolytbrücken am unteren Ende des Meßkopfes eine elektrolytisch leitende Verbindung zur Kathode haben,

   mehreren konzentrisch zu dem Lichtleiterschacht (4) in einer Kontaktfläche am unteren Ende des Meßkopfes endenden Kathodenzuleitungen (8), die mit einer Kathodenfläche (12) verbunden sind, die ihrerseits von einer den unteren Teil des Meßkopfs (1) umschließenden hoch-sauerstoff-durchlässigen Meßflächen-Membran (13) getragen wird, wobei zwischen Meßkopf (1) und Membran (13) ein Raum (14) mit einer alkalischen Lösung eingeschlossen ist,

   b) einem in dem Lichtleiterschacht (4) angeordneten Y-Lichtleiter (2), an dessen Anregungsschenkel (15) eine Lichtquelle (16) und an dessen Detektionsschenkel (18) ein Lichtmeßgerät (20) angeordnet ist und

   c) einem in dem die Anode (9) und Kathode (8) am oberen Ende des Meßkopfes verbindenden Stromkreis angeordneten Meßgerät (22).

2. Vorrichtung nach Anspruch 1, dadurch gekennzeichnet, daß die Kathodenfläche (12) aus Silber und die Anode (9) aus Blei besteht und die Elektrolytbrücken und die alkalische Lösung eine Kaliumhydroxydlösung sind.

3. Vorrichtung nach einem der Ansprüche 1 oder 2, dadurch gekennzeichnet, daß die lichtdichte Deckmembran (11) und die Meßflächen-Membran (13) aus hoch sauerstoff-durchlässigem Silikonmaterial bestehen.

4. Vorrichtung nach einem der Ansprüche 1 bis 3, dadurch gekennzeichnet, daß zur Messung des angeregten Fluoreszenz-Lichts am Ende des Detektionsschenkels (18) des Y-Lichtleiters (2) ein Filter (19) und hinter diesem ein Fotovervielfacher (20) angebracht ist, dessen Signal, gegebenenfalls über einen Vorverstärker (23) einem Schreiber (24) zugeführt wird.

5. Vorrichtung nach einem der Ansprüche 1 bis 4, dadurch gekennzeichnet, daß im Stromkreis der elektrochemischen Zelle zwischen dem Meßgerät (22) und der Anode (9) ein, gegebenenfalls regelbarer, Widerstand (21) angeordnet ist.

6. Vorrichtung nach einem der Ansprüche 1 bis 5, dadurch gekennzeichnet, daß das Meßsignal des Meßgeräts (22) einem Schreiber (24) zugeführt wird.

7. Vorrichtung nach einem der Ansprüche 1 bis 6, dadurch gekennzeichnet, daß die Lichtquelle (16) ein pulsatiles Licht abgibt.

8. Vorrichtung nach einem der Ansprüche 1 bis 7, dadurch gekannzeichnet, daß das Luminisenzsignal in Form der Abklingzeit fortlaufend bestimmt wird.

Fig. 1

Fig. 2

**Europäisches Patentamt**

# EUROPÄISCHER RECHERCHENBERICHT

Nummer der Anmeldung

EP 96 11 8305

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | WO 92 12705 A (A. MAYEVSKY U. A.)<br>* Seite 6, Zeile 4 - Seite 7, Zeile 15 *<br>* Seite 8, Zeile 25 - Seite 9, Zeile 27 *<br>--- | 1,4,6-8 | A61B5/00 |
| A | GB 2 033 575 A (P. ROLFE)<br>* Seite 3, Zeile 70 - Seite 4, Zeile 54 *<br>--- | 1,6 | |
| A | US 4 041 932 A (M.A. FOSTICK)<br>* Spalte 17, Zeile 8 - Spalte 18, Zeile 6 *<br>--- | 1,7 | |
| A | US 4 259 963 A (A. HUCH)<br>* Spalte 3, Zeile 64 - Spalte 4, Zeile 61 *<br>--- | 1 | |
| A | DE 26 30 468 A (DORNIER SYSTEM GMBH)<br>* Seite 5, Zeile 26 - Seite 8, Zeile 10 *<br>--- | 1 | |
| A | DE 32 31 483 A (A. HUCH)<br>* Seite 6, Zeile 3 - Seite 7, Zeile 15 *<br>----- | 1 | |

RECHERCHIERTE SACHGEBIETE (Int.Cl.6)

A61B

Der vorliegende Recherchenbericht wurde für alle Patentansprüche erstellt

| Recherchenort | Abschlußdatum der Recherche | Prüfer |
|---|---|---|
| DEN HAAG | 21.Juli 1997 | RIEB, D |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
............................................................
& : Mitglied der gleichen Patentfamilie, übereinstimmendes Dokument

EPO FORM 1503 03.82 (P04C03)